# EUROPEAN PATENT APPLICATION

(11) **EP 4 542 210 A1**
(43) Date of publication of application: **23.04.2025**
(21) Application number: 24201132.8
(22) Date of filing: 18.09.2024
(51) Int. Cl.: G01N 27/04, H02H 5/08

(54) **LIQUID INTRUSION DETECTION DEVICE, LIQUID INTRUSION DETECTION METHOD AND COMPUTER SYSTEM THEREOF**

(30) Priority: 20.10.2023 CN 202311364012
(71) Applicant: Getac Technology Corporation, New Taipei City 221009 (TW)
(72) Inventor: Lin, Hsin-Chih, Taipei City, R.O.C. (TW)
(74) Representative: dompatent von Kreisler Selting Werner - Partnerschaft von Patent- und Rechtsanwälten mbB

(57) **Abstract**

A liquid intrusion detection device (10) comprises at least one circuit board (100), a plurality of liquid detection components (101a, 101b), a multiplexer (102) and a computing component (103). The plurality of liquid detection components (101a, 101b) are disposed on the at least one circuit board (100). The multiplexer (102) is disposed on the at least one circuit board (100) and has a plurality of output pins respectively connected to the plurality of liquid detection components (101a, 101b). The computing component (103) is disposed on the at least one circuit board (100) and connected to the multiplexer (102), and is configured to switch the multiplexer (102) to output a detection signal to one of the plurality of liquid detection components (101a, 101b) through one of the plurality of output pins, and output a liquid intrusion notification signal when a response signal of the detection signal meets a preset potential.

## Description

### CROSS-REFERENCE TO RELATED APPLICATIONS

### BACKGROUND

### 1. Technical Field

This disclosure relates to a liquid intrusion detection device, liquid intrusion detection method and computer system thereof.

### 2. Related Art

Currently, for the semi-rugged computers and the fully-rugged computers, the test specifications and the humidity test standards are the same. However, due to the lack of an airtight design in semi-rugged computers, moisture may enter the system and circuit board during the humidity test, and the only way to reduce moisture expose is by attaching mylar to the circuit board. Therefore, the current problem in this field is to find a solution to detect whether moisture has entered the circuit board and may have caused damage to some chips, and to provide timely notification so that the microcontroller/central processor and other computing components of the system can take corresponding action.

### SUMMARY

Accordingly, this disclosure provides a liquid intrusion detection device, liquid intrusion detection method and computer system thereof.

According to one or more embodiments of this disclosure, a liquid intrusion detection device comprises at least one circuit board, a plurality of liquid detection components, a multiplexer and a computing component. The plurality of liquid detection components are disposed on the at least one circuit board. The multiplexer is disposed on the at least one circuit board and has a plurality of output pins respectively connected to the plurality of liquid detection components. The computing component is disposed on the at least one circuit board and connected to the multiplexer, and is configured to switch the multiplexer to output a detection signal to one of the plurality of liquid detection components through one of the plurality of output pins, and output a liquid intrusion notification signal when a response signal of the detection signal meets a preset potential.

According to one or more embodiments of this disclosure, a liquid intrusion detection method comprises: providing a liquid intrusion detection device, wherein the liquid intrusion detection device comprises at least one circuit board and a plurality of liquid detection components, a multiplexer and a computing component disposed on the at least one circuit board; and executing with the computing component: switching the multiplexer to output a detection signal to one of the plurality of liquid detection components; detecting a response signal of the detection signal; and outputting a liquid intrusion notification signal when a response signal of the detection signal meets a preset potential.

According to one or more embodiments of this disclosure, a computer system comprises a power switch and a liquid intrusion detection device as described above. The liquid intrusion detection device is connected to the power switch, wherein the computing component outputs the liquid intrusion notification signal to the power switch to turn off all the power switch.

In view of the above description, the liquid intrusion detection device, liquid intrusion detection method and computer system thereof of the present disclosure, by disposing multiple liquid detection components on one or more circuit boards, may perform water vapor/humidity detection on any selected area of the circuit board without the need to install additional detection components on the circuit board, and may reduce the costs. By using the computing component to switch each pin of the multiplexer, the response signal of the specific liquid detection component may be obtained and a liquid intrusion notification may be issued when it is determined that liquid intrusion has occurred. In this way, one computing component and multiple liquid detection components may be integrated through a multiplexer, without the need for additional computing components such as multiple microcontrollers/central processors.

### BRIEF DESCRIPTION OF THE DRAWINGS

The present disclosure will become more fully understood from the detailed description given hereinbelow and the accompanying drawings which are given by way of illustration only and thus are not limitative of the present disclosure and wherein:
FIG. 1 is a block diagram of a liquid intrusion detection device according to an embodiment of the present disclosure;
FIG. 2 is a block diagram of a liquid intrusion detection device according to another embodiment of the present disclosure;
FIG. 3A is a schematic diagram of the pin connections between the multiplexer and the computing component of the liquid intrusion detection device according to an embodiment of the present disclosure;
FIG. 3B is a schematic diagram of the pin connections between the multiplexer and the computing component of the liquid intrusion detection device according to another embodiment of the present disclosure;
FIG. 4 is a schematic diagram of the liquid detection component of the liquid intrusion detection device according to an embodiment of the present disclosure;
FIG. 5 is a block diagram of a computer system according to an embodiment of the present disclosure;
FIG. 6 is a flow chart of a liquid intrusion detection method according to an embodiment of the present disclosure; and
FIG. 7 is a flow chart of a liquid intrusion detection method according to another embodiment of the present disclosure.

### DETAILED DESCRIPTION

In the following detailed description, for purposes of explanation, numerous specific details are set forth in order to provide a thorough understanding of the disclosed embodiments. According to the description, claims and the drawings disclosed in the specification, one skilled in the art may easily understand the concepts and features of the present invention. The following embodiments further illustrate various aspects of the present invention, but are not meant to limit the scope of the present invention.

The liquid intrusion detection device, liquid intrusion detection method and computer system thereof described herein may be applied to electronic devices or equipment that are sensitive to liquid, humidity or moisture, but the present disclosure is not limited thereto.

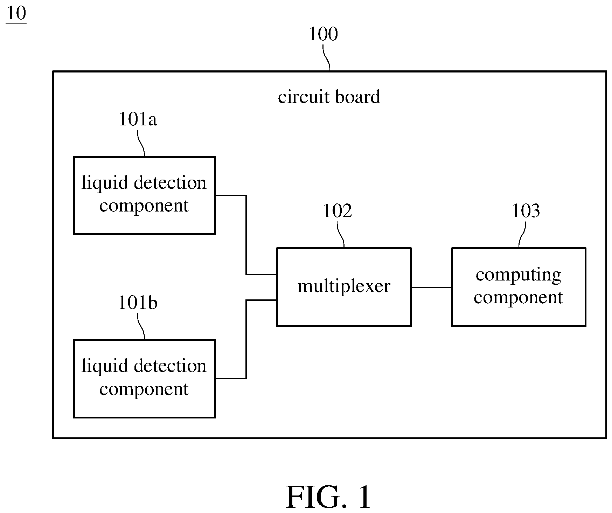

Please refer to FIG. 1 which is a block diagram of a liquid intrusion detection device according to an embodiment of the present disclosure. As shown in FIG. 1, a liquid intrusion detection device 10 comprises at least one circuit board 100, a plurality of liquid detection components 101a and 101b, a multiplexer 102 and a computing component 103. The plurality of liquid detection components 101a and 101b are disposed on the at least one circuit board 100. The multiplexer 102 is disposed on the at least one circuit board 100 and has a plurality of output pins respectively connected to the plurality of liquid detection components 101a and 101b. The computing component 103 is disposed on the at least one circuit board 100 and connected to the multiplexer 102, and is configured to switch the multiplexer 102 to output a detection signal to one of the plurality of liquid detection components 101a and 101b through one of the plurality of output pins, and output a liquid intrusion notification signal when a response signal of the detection signal meets a preset potential.

In the present embodiment, the circuit board 100 of the liquid intrusion detection device 10 may be an existing circuit board of the system, that is, the liquid intrusion detection device 10 may be integrated with the circuit board of any device (such as a personal computer). The liquid detection components 101a and 101b are disposed on the circuit board 100, and are preferably integrated with the circuit board 100 through PCB layout. The number and position of the liquid detection components 101a and 101b may be adjusted according to application requirements. For example, the liquid detection components 101a and 101b may be disposed at each corner of the circuit board 100 to maximize the detection range for liquid intrusion; or, the liquid detection components 101a and 101b may be disposed around a specific chip to ensure that the specific chip is protected from the influence of moisture; or, the liquid detection components 101a and 101b may be disposed adjacent to connectors, such as connection ports, screw holes, etc., so as to be triggered as soon as the liquid intrusion occurs and generate a notification signal.

The multiplexer 102 (MUX) may select one of a plurality of input signals for output. The computing component 103 may be implemented through a microcontroller, a central processing unit (CPU) or a programmable logic controller (PLC). The computing component 103 is connected to the plurality of liquid detection components 101a and 101b through the multiplexer 102 to obtain response signals of each liquid detection components 101a and 101b, and output a liquid intrusion notification signal when determining that the potential of the response signal reaches a preset potential. Specifically, the computing component 103 may periodically switch a plurality of output pins of the multiplexer 102 to output a detection signal to one of the plurality of liquid detection components 101a and 101b through one of the plurality of output pins, and when a response signal of the detection signal meets a preset potential, the computing component 103 may output a liquid intrusion notification signal to provide immediate notification of potential liquid intrusion.

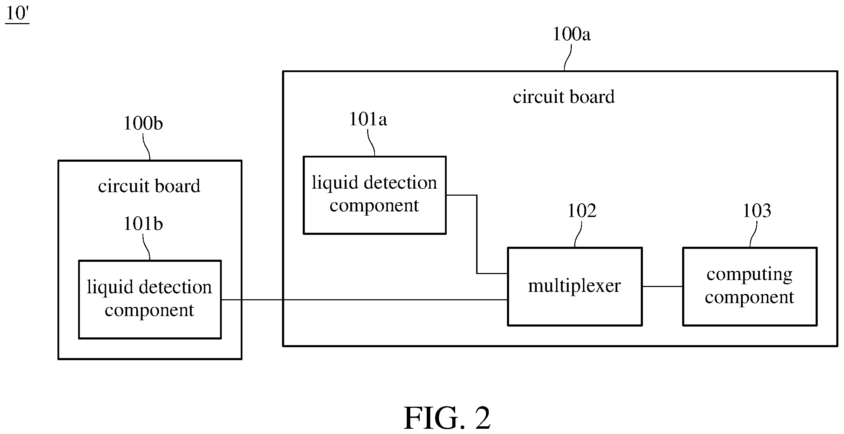

Please refer to FIG. 2 which is a block diagram of a liquid intrusion detection device according to another embodiment of the present disclosure. As shown in FIG. 2, the liquid detection components 101a and 101b of the liquid intrusion detection device 10' in the present embodiment are respectively disposed on two circuit boards 100a and 100b, wherein the two circuit boards 100a and 100b may be connected to each other through a flexible PCB (FPCB), a cable or a board-to-board connector. The liquid detection component 101b may be electrically connected to the multiplexer 102 on another circuit board 100a through the connection method described above. In the present embodiment, other descriptions about the operations of the multiplexer 102 and the computing component 103 are basically the same as those in the embodiment of FIG. 1 and are omitted herein.

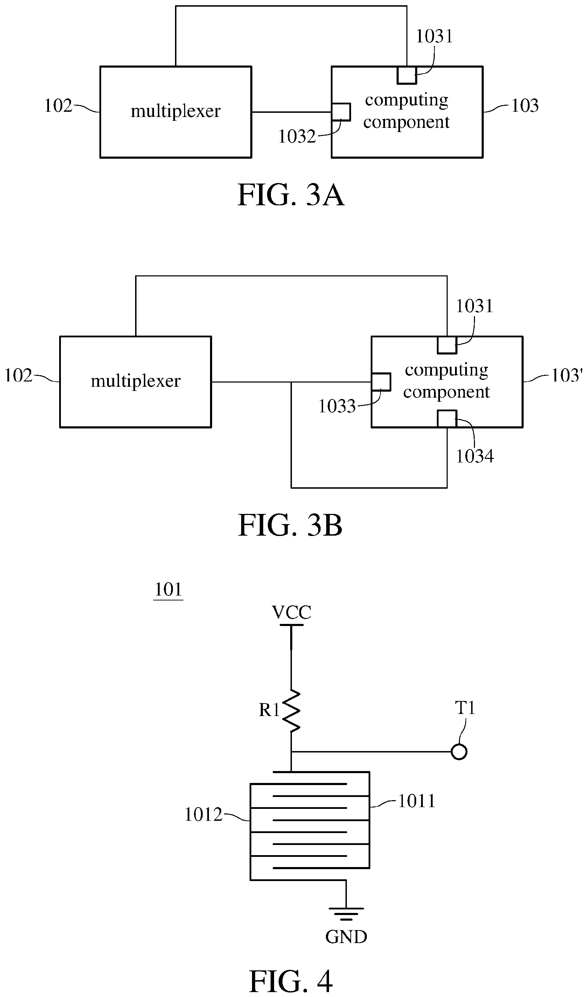

Please refer to FIG. 3A which is a schematic diagram of the pin connections between the multiplexer and the computing component of the liquid intrusion detection device according to an embodiment of the present disclosure. As shown in FIG. 3A, in the present embodiment, the computing component 103 has a switching signal output pin 1031 and a detection pin 1032 that are connected to the multiplexer 102. The switching signal output pin 1031 is configured to output a switching signal to the multiplexer 102, and the detection pin 1032 is configured to output the detection signal and detect the response signal. Specifically, the computing component 103 in the present embodiment may transmit a switching signal to the multiplexer 102 through the switching signal output pin 1031, and output the detection signal to the multiplexer 102 through the detection pin 1032, wherein the switching signal specifies one of the plurality of liquid detection components. When the multiplexer 102 receives the switching signal, the multiplexer 102 may output the received detection signal to the specified liquid detection component through the corresponding output pin, and then obtain the response signal of the specified liquid detection component. Then, the computing component 103 may obtain the response signal through the detection pin 1032, that is, obtain a liquid detection result of the specific liquid detection component. Therefore, in the present embodiment, the pin for outputting the detection signal and the pin for receiving the response signal of the computing component 103 are the same pin (detection pin 1032).

Please refer to FIG. 3B which is a schematic diagram of the pin connections between the multiplexer and the computing component of the liquid intrusion detection device according to another embodiment of the present disclosure. As shown in FIG. 3B, in the present embodiment, the computing component 103' has a switching signal output pin 1031, a detection signal output pin 1033 and a response signal input pin 1034 that are connected to the multiplexer 102. The switching signal output pin 1031 is configured to output a switching signal to the multiplexer 102, the detection signal output pin 1033 is configured to output the detection signal, and the response signal input pin 1034 is connected to the detection signal output pin 1033 to detect the response signal. Specifically, the computing component 103' in the present embodiment may transmit a switching signal to the multiplexer 102 through the switching signal output pin 1031, and output the detection signal to the multiplexer 102 through the detection signal output pin 1033, wherein the switching signal corresponds to the plurality of liquid detection components. When the multiplexer 102 receives the switching signal, the multiplexer 102 may output the received detection signal to a specified liquid detection component through the corresponding output pin, and then obtain the response signal of the specified liquid detection component. Then, the computing component 103 may obtain the response signal through the response signal input pin 1034, that is, obtain a liquid detection result of the specified liquid detection component. Therefore, in the present embodiment, although the multiplexer 102 obtains the detection signal of the computing component 103' and transmits the response signal to the computing component 103' through one pin, the computing component 103' outputs the detection signal (through detection signal output pin 1033) and obtains a response signal (through response signal input pin 1034) through different pins.

Please refer to FIG. 4 which is a schematic diagram of the liquid detection component of the liquid intrusion detection device according to an embodiment of the present disclosure. As shown in FIG. 4, one of the plurality of liquid detection components 101 comprises a first comb-shape electrode 1011 and a second comb-shape electrode 1012 that are disposed correspondingly, wherein the first comb-shape electrode 1011 is connected to the multiplexer and is configured to receive an operating voltage VCC, and the second comb-shape electrode 1012 is configured to be connected to the ground terminal GND. Specifically, the first comb-shape electrode 1011 and the second comb-shape electrode 1012 may be formed on the circuit board of the liquid intrusion detection device. The first comb-shape electrode 1011 is connected to an operating voltage VCC through a resistor R1, wherein the operating voltage VCC may be, for example, 3.3 volts, and the first comb-shape electrode 1011 is connected to one end T1 and connected to the multiplexer through the one end T1. The second comb-shape electrode 1012 is grounded and is electrically isolated from the first comb-shape electrode 1011 when there is no moisture around. When there is moisture around and the moisture is condensed into tiny droplets, the second comb-shape electrode 1012 and the first comb-shape electrode 1011 have a certain degree of electrical conduction.

In this way, when the computing component transmits a detection signal to the multiplexer, the multiplexer may obtain a response signal with a corresponding voltage value through the one terminal T1 and transmit the response signal back to the computing component. If the voltage value of the response signal is close to the operating voltage VCC, the computing component determines that the first comb-shape electrode 1011 and the second comb-shape electrode 1012 are electrically isolated from each other; if the voltage value of the response signal is lower than the operating voltage VCC to a certain extent, then the computing component determines that the first comb-shape electrode 1011 and the second comb-shape electrode 1012 are electrically connected to each other, and outputs a liquid intrusion notification signal.

Furthermore, in one implementation, the detection signal output by the computing component through the output pin by switching the multiplexer may be a pulse-width modulation (PWM) signal, to reduce the power consumption of computing component when performing detection. In this implementation, if the response signal corresponds to the pulse width modulation signal (the voltage value corresponds to the operating voltage VCC), the computing component determines that the first comb-shape electrode 1011 and the second comb-shape electrode 1012 are electrically isolated from each other; if the voltage value of the response signal is lower than the operating voltage VCC to a certain extent, the computing component determines that the first comb-shape electrode 1011 and the second comb-shape electrode 1012 are electrically connected to each other, and outputs a liquid intrusion notification signal.

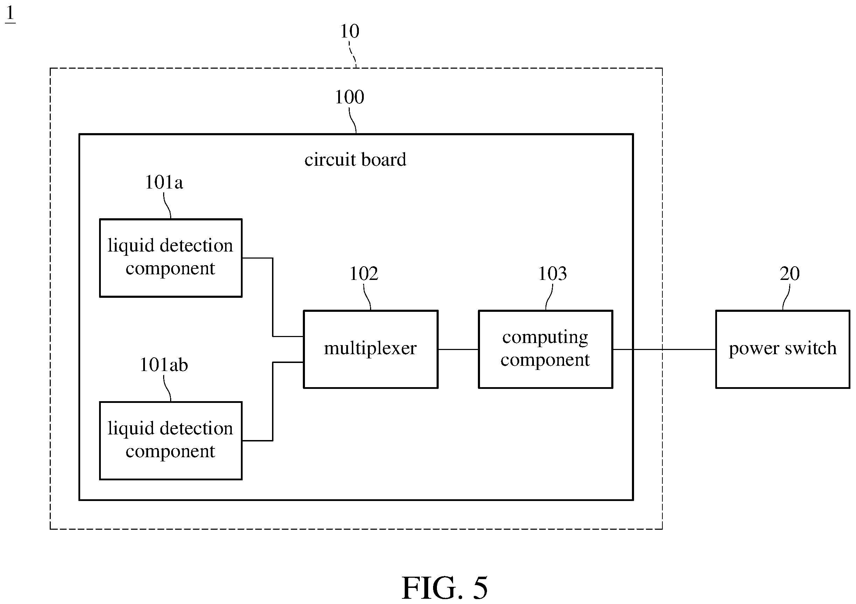

Please refer to FIG. 5 which is a block diagram of a computer system according to an embodiment of the present disclosure. As shown in FIG. 5, the computer system 1 comprises a liquid intrusion detection device 10 and a power switch 20. The liquid intrusion detection device 10 comprises at least one circuit board 100, a plurality of liquid detection components 101a and 101b, a multiplexer 102 and a computing component 103. The plurality of liquid detection components 101a and 101b are disposed on the at least one circuit board 100. The multiplexer 102 is disposed on the at least one circuit board 100 and has a plurality of output pins respectively connected to a plurality of liquid detection components 101a and 101b. The computing component 103 is disposed on the at least one circuit board 100 and connected to the multiplexer 102, and is configured to switch the multiplexer 102 to output a detection signal to one of the plurality of liquid detection components 101a and 101b through one of the plurality of output pins, and output a liquid intrusion notification signal when a response signal of the detection signal meets a preset potential. The liquid intrusion detection device 10 is connected to the power switch 20, wherein the computing component 103 outputs the liquid intrusion notification signal to the power switch 20 to turn off the power switch 20.

The liquid intrusion detection device 10 of the present embodiment is basically the same as the liquid intrusion detection device 10 of the embodiment shown in FIG. 1, and may be combined with the embodiments described above, and repeated descriptions are omitted herein. In present embodiment, the computing component 103 of the liquid intrusion detection device 10 is specifically connected to a power switch 20, where the power switch 20 may be used to control the power supply of the system. When the computing component 103 determines that liquid intrusion is detected, the computing component 103may output a liquid intrusion notification signal to the power switch 20 and control the power switch 20 to be turn off to avoid damage caused by liquid intrusion. Specifically, the power switch 20 may be formed by using an N-type transistor (N MOSFET) or a P-type transistor (P MOSFET) or a combined power switch integrated chip, or a circuit with a power switching function formed by the N-type transistor and P-type transistor described above alone or in combination. For example, the power switch 20 may be a power switch chip with a model number of XC8107AC20ER-G, or may be a switching circuit designed by using a P-type transistor with a model number of MDV3604 and an N-type transistor with a model number of L2N7002WT1G. In addition, other types of switch circuits with power-off or isolation circuit designs may also be used to achieve the above effects.

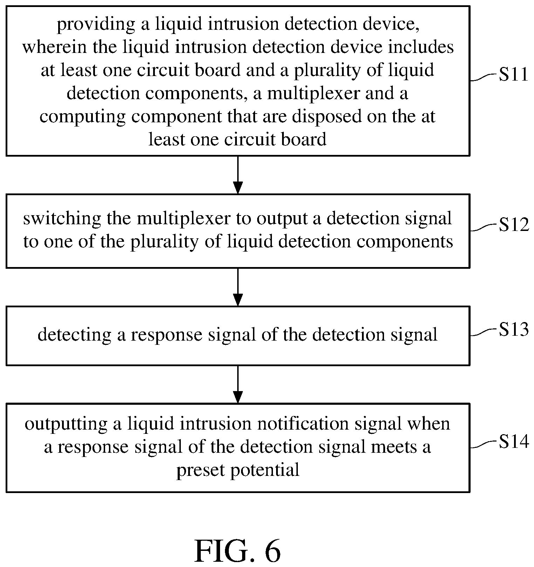

Please refer to FIG. 6 which is a flow chart of a liquid intrusion detection method according to an embodiment of the present disclosure. As shown in FIG. 6, a liquid intrusion detection method comprises: step S11: providing a liquid intrusion detection device, wherein the liquid intrusion detection device comprises at least one circuit board and a plurality of liquid detection components, a multiplexer and a computing component that are disposed on the at least one circuit board; and executing with the computing component: step S12: switching the multiplexer to output a detection signal to one of the plurality of liquid detection components; step S 13: detecting a response signal of the detection signal; and step S14: outputting a liquid intrusion notification signal when a response signal of the detection signal meets a preset potential.

In step S11, the provided liquid intrusion detection device may refer to the liquid intrusion detection device in the embodiment of FIGs. 1 to 5. Please refer to FIGs. 1, 3 and 5 altogether, in step S12, the computing component 103 may switch the multiplexer 102 to output the detection signal to one of the liquid detection components 101a and 101b by switching the signal output pin 1031. In step S13, the computing component 103 may detect the voltage level of the response signal to determine whether liquid intrusion occurs near the corresponding liquid detection component. In step S14, when the computing component 103 determines that the voltage level of the response signal meets a preset potential (lower than the operating voltage VCC to a certain extent), the computing component 103 may output a liquid intrusion notification signal.

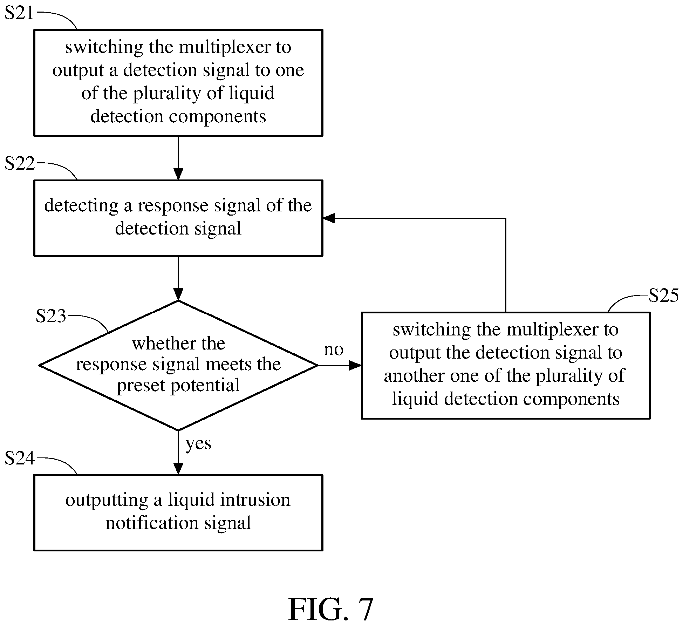

Please refer to FIG. 7 which is a flow chart of a liquid intrusion detection method according to another embodiment of the present disclosure. As shown in FIG. 7, in addition to executing step S11 to S14 shown in FIG. 6, the liquid intrusion detection method of the present embodiment may further execute with the computing component: step S21: switching the multiplexer to output a detection signal to one of the plurality of liquid detection components; step S22: detecting a response signal of the detection signal; step S23: determining whether the response signal meets the preset potential; if the determination result is "yes", execute step S24: outputting a liquid intrusion notification signal; if the determination result is "no", execute step S25: switching the multiplexer to output the detection signal to another one of the plurality of liquid detection components.

Steps S21 and S22 in the present embodiment are basically the same as steps S12 and S13 shown in FIG. 6, and repeated descriptions are omitted herein. Please refer to FIGs. 1, 3 and 5 altogether, in step S23, the computing component 103 may determine whether the response signal meets a preset potential, wherein the preset potential may correspond to a specific proportional value of the operating voltage VCC. In step S24, when the computing component 103 determines that the response signal meets the preset potential (lower than the operating voltage VCC to a certain extent), the computing component 103 may output a liquid intrusion signal. In step S25, when the computing component 103 determines that the response signal does not meet the preset potential (not lower than the operating voltage VCC to a certain extent), the computing component 103 may switch the multiplexer 102 to output the detection signal to another one of the plurality of liquid detection components, that is, re-executes step S22 on another liquid detection component.

In view of the above description, the liquid intrusion detection device, liquid intrusion detection method and computer system thereof of the present disclosure, by disposing multiple liquid detection components on one or more circuit boards, may perform water vapor/humidity detection on any selected area of the circuit board without the need to install additional detection components on the circuit board, and may reduce the costs. By using the computing component to switch each pin of the multiplexer, the response signal of the specific liquid detection component may be obtained and a liquid intrusion notification may be issued when it is determined that liquid intrusion has occurred. In this way, one computing component and multiple liquid detection components may be integrated through a multiplexer, without the need for additional computing components such as multiple microcontrollers/central processors. In addition, by using pulse width modulation as the detection signal of the computing component, the power consumption of the computing component when performing detection may be reduced.

## Claims

1. A liquid intrusion detection device (10), comprising:
at least one circuit board (100);
a plurality of liquid detection components (101a, 101b) disposed on the at least one circuit board (100);
a multiplexer (102) disposed on the at least one circuit board (100) and having a plurality of output pins respectively connected to the plurality of liquid detection components (101a, 101b); and
a computing component (103) disposed on the at least one circuit board (100) and connected to the multiplexer (102), and configured to switch the multiplexer (102) to output a detection signal to one of the plurality of liquid detection components (101a, 101b) through one of the plurality of output pins, and output a liquid intrusion notification signal when a response signal of the detection signal meets a preset potential.

2. The liquid intrusion detection device (10) of claim 1, wherein the detection signal is a pulse width modulation signal.

3. The liquid intrusion detection device (10) of claim 1, wherein the computing component (103) has a switching signal output pin (1031) and a detection pin (1032) that are connected to the multiplexer (102), the switching signal output pin (1031) is configured to output a switching signal to the multiplexer (102), and the detection pin (1032) is configured to output the detection signal and detect the response signal.

4. The liquid intrusion detection device (10) of claim 1, wherein the computing component (103) has a switching signal output pin (1031), a detection signal output pin (1033) and a response signal input pin (1034) that are connected to the multiplexer (102), the switching signal output pin (1031) is configured to output a switching signal to the multiplexer (102), the detection signal output pin (1033) is configured to output the detection signal, and the response signal input pin (1034) is connected to the detection signal output pin (1033) to detect the response signal.

5. The liquid intrusion detection device (10) of claim 1, wherein one of the plurality of liquid detection components (101a, 101b) comprises a first comb-shape electrode (1011) and a second comb-shape electrode (1012) that are disposed correspondingly, the first comb-shape electrode (1011) is connected to the multiplexer (102) and is configured to receive an operating voltage (VCC), and the second comb-shape electrode (1012) is configured to be connected to a ground terminal.

6. The liquid intrusion detection device (10) of claim 1, wherein the at least one circuit board (100) comprises a plurality of circuit boards (100a, 100b), and two of the plurality of liquid detection components (101a, 101b) are disposed on different circuit boards (100a, 100b) among the plurality of circuit boards (100a, 100b).

7. A computer system, comprising:
a power switch (20); and
a liquid intrusion detection device (10) comprising at least one circuit board (100);
a plurality of liquid detection components (101a, 101b) disposed on the at least one circuit board (100);
a multiplexer (102) disposed on the at least one circuit board (100) and having a plurality of output pins respectively connected to the plurality of liquid detection components (101a, 101b); and
a computing component (103) disposed on the at least one circuit board (100) and connected to the multiplexer (102), and configured to switch the multiplexer (102) to output a detection signal to one of the plurality of liquid detection components (101a, 101b) through one of the plurality of output pins, and output a liquid intrusion notification signal when a response signal of the detection signal meets a preset potential, wherein the liquid intrusion detection device (10) is connected to the power switch (20), and the computing component (103) outputs the liquid intrusion notification signal to the power switch (20) to turn off the power switch (20).

8. The computer system of claim 7, wherein the detection signal is a pulse width modulation signal.

9. The computer system of claim 7, wherein the computing component (103) has a switching signal output pin (1031) and a detection pin (1032) that are connected to the multiplexer (102), the switching signal output pin (1031) is configured to output a switching signal to the multiplexer (102), and the detection pin (1032) is configured to output the detection signal and detect the response signal.

10. The computer system of claim 7, wherein the computing component (103) has a switching signal output pin (1031), a detection signal output pin (1033) and a response signal input pin (1034) that are connected to the multiplexer (102), the switching signal output pin (1031) is configured to output a switching signal to the multiplexer (102), the detection signal output pin (1033) is configured to output the detection signal, and the response signal input pin (1034) is connected to the detection signal output pin (1033) to detect the response signal.

11. The computer system of claim 7, wherein one of the plurality of liquid detection components (101a, 101b) comprises a first comb-shape electrode (1011) and a second comb-shape electrode (1012) that are disposed correspondingly, the first comb-shape electrode (1011) is connected to the multiplexer (102) and is configured to receive an operating voltage (VCC), and the second comb-shape electrode (1012) is configured to be connected to a ground terminal.

12. The computer system of claim 7, wherein the at least one circuit board (100) comprises a plurality of circuit boards (100a, 100b), and two of the plurality of liquid detection components (101a, 101b) are disposed on different circuit boards (100a, 100b) among the plurality of circuit boards (100a, 100b).

13. A liquid intrusion detection method, comprising:
providing a liquid intrusion detection device (10), wherein the liquid intrusion detection device (10) comprises at least one circuit board (100) and a plurality of liquid detection components (101a, 101b), a multiplexer (102) and a computing component (103) that are disposed on the at least one circuit board (100); and
executing with the computing component (103):
switching the multiplexer (102) to output a detection signal to one of the plurality of liquid detection components (101a, 101b);
detecting a response signal of the detection signal; and
outputting a liquid intrusion notification signal when a response signal of the detection signal meets a preset potential.

14. The liquid intrusion detection method of claim 13, wherein the detection signal is a pulse width modulation signal.

15. The liquid intrusion detection method of claim 13, further comprising executing with the computing component (103):
switching the multiplexer (102) to output the detection signal to another one of the plurality of liquid detection components (101a, 101b);
detecting another response signal of the detection signal; and
outputting another liquid intrusion notification signal when said another response signal of the detection signal meets the preset potential.
